# EUROPEAN PATENT SPECIFICATION

(11) **EP 2 490 994 B1**
(45) Date of publication and mention of the grant of the patent: **04.03.2015**
(21) Application number: 10765799.1
(22) Date of filing: 18.10.2010
(51) Int. Cl.: H01F 27/28, H01F 27/32

(54) **TRANSFORMER**
TRANSFORMATOR
TRANSFORMATEUR

(30) Priority: 19.10.2009 EP 09173371
(43) Date of publication of application: 29.08.2012
(73) Proprietor: ABB Technology AG, 8050 Zürich (CH)
(72) Inventor: MURILLO, Rafael, E-50004 Zaragoza (ES)
(74) Representative: ABB Patent Attorneys
(86) International application number: PCT/EP2010/065608
(87) International publication number: WO 2011/048039

(56) References cited:
- EP-A2- 1 733 764
- FR-A- 1 070 676
- US-A- 4 488 134
- US-A- 4 523 171
- US-A- 6 157 282
- US-A1- 2005 263 737
- US-A1- 2008 033 201
- US-A1- 2009 183 856

## Description

The present invention relates to a transformer, according to the preamble of claim 1, and to the use of the transformer at a voltage in the range from 10 to 300 kV, preferably from 20 to 150 kV, more preferably from 36 to 110 kV and most preferably at about 72.5 kV, according to claim 13.

Transformers are well known in the art and designate devices that transfer electrical energy from one circuit to another through inductively coupled conductors, i.e. the transformer windings. In general, the windings are wound around a magnetic core. A current in the first ("primary") winding creates a magnetic field in the magnetic core, said magnetic field inducing a voltage in the second ("secondary") winding. This effect is called mutual induction.

The components of the electrical active part, which includes the windings and the magnetic core, must be insulated from each other depending on the dielectric requirements between them.

With regard to the insulation, two types of transformers can be distinguished:
In a liquid or gas transformer, on the one hand, the electrical active part comprising the windings and the magnetic core is arranged in a tank or vessel, which is filled with a dielectric insulation medium having higher insulating properties than air. Specifically, the insulation medium is a liquid, such as oil, silicone or midel, in a liquid-insulated transformer, and a gas, such as SF₆ or N₂ either at atmospheric or elevated pressure, in a gas-insulated transformer. A water cooled transformer is known from FR-1070676.

A dry transformer, on the other hand, is devoid of a tank, and the electrical active part is thus surrounded by air at atmospheric pressure.

For a voltage higher than 36 kV, gas or liquid transformers are typically used. Due to the relatively high insulating performance of the insulation medium, the clearance between the components of the electrical active part is relatively small. These transformers have however the disadvantage that they are relatively complex and - due to the use of a tank and of an insulation medium different than air - relatively costly. Also, cooling means are often required in such transformers in order to actively cool the insulating space, and in particular the space between the windings. These cooling means further contribute to the complexity and the high cost of gas and liquid transformers.

Dry transformers, which are typically used up to a voltage of 36 kV, are less complex, but have the disadvantage of big clearances due to the relatively low insulating performance of air. Thus, the spatial requirements of a dry transformer are relatively demanding and its weight is relatively high. Due to the amount of material required, dry transformers are thus also relatively costly.

Based on the above disadvantages of the state of the art, the object of the present invention is to provide a transformer, which - even if designated for relatively high voltages - allows for a very simple and compact design and at the same time also for a substantial decrease in the manufacturing cost.

The object is solved by the transformer according to claim 1. Preferred embodiments of the transformer are given in the dependent claims.

The transformer of the present invention comprises a block, which defines a compartment closed and separated from the remaining insulating space of the transformer. Said closed compartment is at least partially arranged within the space between the windings, i.e. within the winding space.

The transformer is characterized in that the closed compartment comprises an insulation fluid which is different from the insulation fluid comprised in the remaining insulating space of the transformer, the insulation fluid comprised in the closed compartment having a higher dielectric breakdown field strength than the insulation fluid comprised in the remaining insulating space.

The present invention makes use of the finding that the clearance between the windings has a much higher impact on the overall manufacturing cost of the transformer than for example the clearance between the high voltage winding and the magnetic core. Specifically, the clearance between the windings is related to the transformer's impedance, which has an impact on the number of turns of the windings and on the cross section area of the magnetic core. In general, the windings of the transformer according to the present invention are circular windings.

Due to the presence of a closed compartment, an insulating fluid having a particularly high dielectric performance, in particular a high dielectric breakdown field strength, can be provided selectively where the dielectric performance is of major importance, i.e. in the winding space. Thus, the clearance between the windings is according to the present invention significantly reduced, which goes along with a lower impedance of the transformer, a higher number of turns in the windings, a reduction in the cross sectional area of the magnetic core, and ultimately also in a reduction of the manufacturing cost.

In the remaining insulation space outside the closed compartment, air at atmospheric pressure can be used without significantly increasing the weight of the transformer, and hence the spatial dimensions of the transformer are increased only moderately and a compact transformer can be achieved without requiring a tank.

Thus, compared to oil or gas insulated conventional transformers, economisation is according to the invention achieved by eliminating the cost of a tank and of a major part of the insulation medium. Compared to dry conventional transformers, economisation is according to the invention achieved by decreasing the amount of material required for the magnetic core. The economic impact of this second aspect is quite substantial, which is emphasized by the fact that in conventional dry transformers the material cost of the magnetic core contributes to about one half of the overall cost of the transformer.

Due to the high insulating performance in the winding space, the transformer of the present invention can be used up to relatively high voltages.

Thus, the present invention combines the advantages of a dry transformer with the advantages of a liquid or gas transformer.

According to a preferred embodiment, the closed compartment corresponds at least approximately to the winding space.

The insulation fluid used in the closed compartment and the remaining insulating space of the transformer according to the present invention can be in liquid or gaseous phase. It is also thinkable that the insulation fluid forms a two-phase system comprising a first part in liquid and a second part in gaseous phase.

For example, the insulation fluid comprised in the closed compartment can be an insulation gas comprising or essentially consisting of SF₆ or air at high pressure or any other insulation gas having a high dielectric performance, in particular having a high dielectric breakdown field strength. Alternatively, the insulation fluid can be an insulation liquid, for example selected from the group consisting of Midel 7131® (M&I Materials Limited), silicone oil and mineral oil.

According to a further preferred embodiment, the closed compartment comprises an insulation gas which has a higher pressure than the insulation gas comprised in the remaining space of the transformer. This is favourable due to the fact that the dielectric performance of the insulation fluid can be enhanced by increasing its pressure. For example, air at an absolute pressure of 5 atm (506625 Pa) has a dielectric strength which is about 3 times higher than in air at atmospheric pressure, so that it is possible to reduce the clearance between the primary (high voltage) and the secondary (low voltage) winding by about 60% to 70%.

Apart from air, an insulation gas selected from the group of N₂, CO₂, SF₆, or an insulation gas mixture selected from the group of SF₆ in CO₂ (the molar ratio of SF₆ typically being about 1%) or of SF₆ in N₂ (the molar ratio of SF₆ typically being about 5% to 10%) have been found particularly well suited if used at an absolute pressure in a range of about 2 atm (202650 Pa) to 5 atm (506625 Pa).

According to a particularly preferred embodiment, the insulation fluid comprises a fluoroketone having from 4 to 12 carbon atoms. These fluoroketones have surprisingly been found to have high insulation capabilities or insulation performance, in particular a high dielectric strength (or breakdown field strength), and at the same time an extremely low global warming potential (GWP).

In general, the fluoroketone according to this preferred embodiment has the general structure

R1-CO-R2

wherein R1 and R2 are at least partially fluorinated chains, said chains being independently from each other linear or branched and having from 1 to 10 carbon atoms. The definition encompasses both perfluorinated ketones as well as hydrofluorinated ketones.

More preferably, the fluoroketone has 6 carbon atoms (also referred to as a C6-fluoroketone). As mentioned above, said C6-fluoroketone can be a perfluorinated ketone (having the molecular formula C₆F₁₂O) or a hydrofluorinated ketone.

If an insulation gas is used, it can be a gas mixture, which preferably comprises the above mentioned fluoroketone together with air or at least one air component, in particular selected from the group consisting of carbon dioxide (CO₂), oxygen (O₂) and nitrogen (N₂), as buffer or carrier gas. Alternatively, the insulation gas can substantially consist of fluoroketone.

Among the most preferred fluoroketones having 6 carbon atoms, dodecafluoro-2-methylpentan-3-one has been found to be particularly preferred for its high insulating properties and its extremely low GWP.

Dodecafluoro-2-methylpentan-3-one (also named 1,1,1,2,2,4,5,5,5-nonafluoro-4-(trifluoromethyl)-3-pentanone, perfluoro-2-methyl-3-pentanone or CF₃CF₂C(O)CF(CF₃)₂) has previously only been considered useful for completely different applications, namely the processing of molten reactive metals (as referred to in WO 2004/090177), for the cleaning of a vapour reactor (as referred to in WO 02/086191) and in fire extinction systems, or in liquid form for cooling of electronic systems, or for the Rankine-process in small power plants (as referred to in EP-A-1764487). Dodecafluoro-2-methylpentan-3-one is clear, colorless and almost odourless. Its structural formula is given in the following:

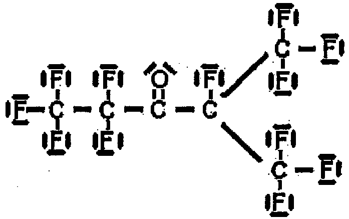

Dodecafluoro-2-methylpentan-3-one has an average lifetime in the atmosphere of about 5 days and its GWP is only about 1. In addition, its ozone depletion potential (ODP) is zero. Thus, the environmental load is much lower than the one of typical conventional insulation gases.

In addition, dodecafluoro-2-methylpentan-3-one is nontoxic and offers outstanding margins of human safety.

Dodecafluoro-2-methylpentan-3-one has a boiling point of 49.2°C at 1 bar. Its vapour pressure, i.e. the pressure of the vapor in equilibrium with its non-vapor phases, is about 40 kPa at 25°C. Thus, a partial pressure of dodecafluoro-2-methylpentan-3-one sufficient for providing excellent dielectric properties can be achieved even at relatively low temperatures.

According to a further preferred embodiment, the windings are embedded in the material of the block. In this embodiment, the material of the block further contributes to the insulation between the windings.

The permittivity of the material of the block is preferably higher than the permittivity of the insulation fluid comprised in the closed compartment. More preferably, the permittivity of the material of the block is higher by a factor of at least 1.5, and more preferably by a factor higher than 3.

Due to the lower permittivity of the insulation fluid in the closed compartment compared to the permittivity of the material of the block, the dielectric stress in the block material and discharge phenomena are reduced, and thus very low. Thus, the dielectric reliability and the lifetime of the block material are improved and are independent on its quality due to the absence of discharge phenomena.

According to a particularly preferred embodiment, the block is preferably made of an epoxy resin. The epoxy resin has a relative permittivity of about 4.3, which is thus much higher than for example in air (having a relative permittivity of 1) and dodecafluoro-2-methylpentan-3-one (having a relative permittivity of 1.84).

In order to further improve the dielectric performance achieved in the winding space, the closed compartment is according to a further preferred embodiment divided by at least one barrier into at least two sub-compartments.

According to a further preferred embodiment, the transformer further comprises a pressure control system designated to control the pressure in the closed compartment. Variations in the pressure caused by for example gas leakage or a change in the temperature can be efficiently compensated for by means of the pressure control system.

In order to allow the block to be cooled, it is further preferred that the transformer further comprises a cooling system, said cooling system comprising means for circulating the insulation fluid in a closed circuit from the closed compartment to an external space where heat is released. This is particularly preferred for embodiments in which heating is an issue, because the winding space cannot be cooled by convection of the insulation fluid as in conventional dry and gas/liquid transformers mentioned above. In particular, the cooling system comprises a pump which allows the insulation fluid to circulate between the closed compartment and an external cooling space, in which the insulation fluid is cooled for example by means of an external heat exchanger.

The transformer of the present invention is particularly suitable for use at a voltage in the range from 10 kV to 300 kV, preferably from 20 kV to 150 kV, more preferably from 36 kV to 110 kV, and most preferably at about 72.5 kV. This is due to the fact that in the ranges mentioned, a maximum reduction of material required and thus a corresponding saving in cost can be achieved.

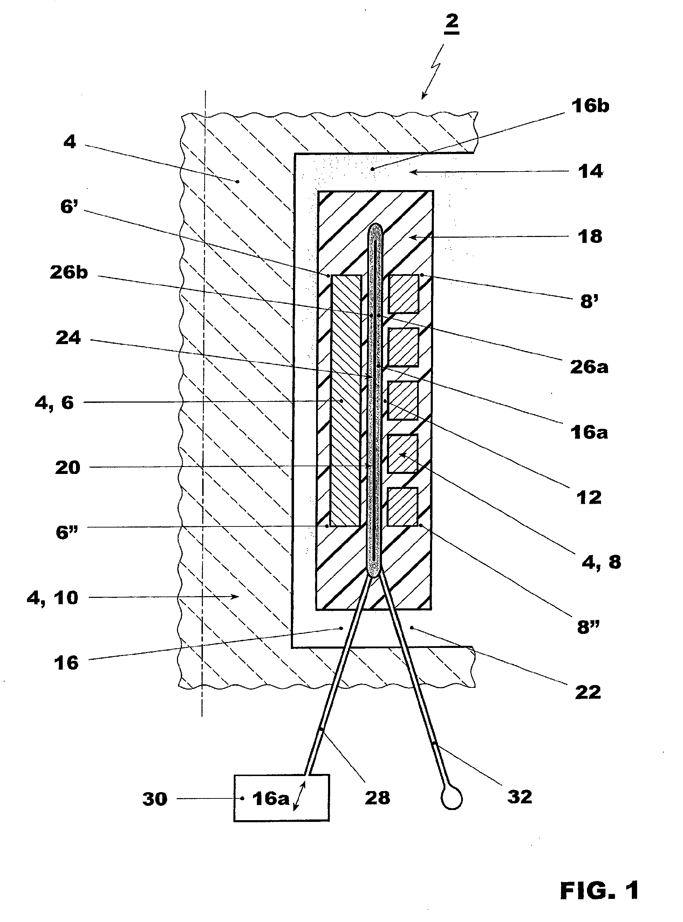

The present invention is further illustrated by way of Fig. 1, which schematically shows a sectional view on a part of a transformer according to the present invention.

The transformer 2 according to Fig. 1 comprises an electrical active part 4, which comprises a primary winding 8, a secondary winding 6 and a magnetic core 10 as electrical active part components.

The windings 6, 8 are wound around the magnetic core 10 and are radially spaced apart from each other by a winding space 12. In the embodiment given in Fig. 1, the radially inner, secondary winding 6 is a low voltage winding and the radially outer, primary winding 8 is a high voltage winding.

The electrical active part 4 is arranged in an insulating space 14 which comprises an insulation fluid 16.

According to Fig. 1, the windings 6, 8 are embedded in a block 18 made of an epoxy resin, said block 18 defining a closed compartment 20 separated from the remaining insulating space 22. This closed compartment 20 is arranged between the windings 6, 8 and thus within the winding space 12. In the embodiment shown in Fig. 1, the closed compartment 20 has an elongate form and with its ends protrudes the upper ends 6', 8' and the lower ends 6", 8" of the windings 6, 8. In the closed compartment 20, a longitudinally extending barrier 24 is arranged, dividing the closed compartment 20 into two sub-compartments 26a, 26b. The solid barrier 24 shall prevent charged components of the insulating fluid 16a to be accelerated electrically in the closed compartment 20 and to induce any flashover.

The fact that the closed compartment 20 arranged within the winding space 12 is completely separated from the remaining insulating space 22 allows the closed compartment 20 to comprise an insulation fluid 16a being different from the insulation fluid 16b of the remaining insulating space 22 and/or having a different pressure. Thus, a specifically high dielectric strength can be provided selectively in the winding space 12.

The electrical active part can be enclosed by a tank (not shown), such as in a conventional liquid or gas transformer, and thus be arranged in a closed insulating space. Alternatively, the transformer 2 can be devoid of a tank, such as in a conventional dry transformer. The insulation fluid in the remaining insulating space 22 outside the closed compartment 20 is in this latter embodiment air at atmospheric pressure.

Fig. 1 also shows schematically a cooling system 28 adapted for cooling the block 18. The cooling system 28 shall preferably comprise means for circulating the insulation fluid 16a in a closed circuit from the closed compartment 20 to an external space 30 where heat is released. This is particularly preferred for embodiments in which heating is an issue, because the winding space 12 cannot be cooled by convection of the insulation fluid 16 or 16a, 16b as in conventional dry and gas/liquid transformers. In particular, the cooling system 28 may comprise a pump which allows the insulation fluid 16a comprised in the otherwise closed compartment 20 to circulate between the closed compartment 20 and the external cooling space 30, in which the insulation fluid 16a is cooled for example by means of an external heat exchanger.

Fig. 1 furthermore shows schematically an embodiment with the transformer 2 comprising a pressure control system 32 designated to control the pressure in the closed compartment 20. Variations in the pressure caused by for example gas leakage or a change in the temperature can be efficiently compensated for by means of the pressure control system 32.

### List of reference numbers

- 2: transformer
- 4: electrical active part
- 6: secondary winding
- 8: primary winding
- 10: magnetic core
- 12: winding space, winding interstitial space
- 14: insulating space
- 16: insulation fluid
- 16a: insulation fluid comprised in closed compartment
- 16b: insulation fluid comprised in remaining insulating space
- 18: block
- 20: closed compartment
- 22: remaining insulating space
- 24: barrier
- 26a, 26b: sub-compartments
- 28: cooling system
- 30: external space, heat exchange or cooling space
- 32: pressure control system.

## Claims

1. A transformer (2) containing an insulating space, which comprises an insulation fluid (16, 16b), and an electrical active part,
the electrical active part being arranged in the insulating space and comprising a primary winding (8), a secondary winding (6) and a magnetic core (10),
said windings (6, 8) being wound around the magnetic core (10) and being radially spaced apart from each other by a winding interstitial space (12),
wherein the transformer (2) further comprises a block (18) defining a compartment (20) closed and separated from a remaining insulating space (22), said closed compartment (20) being at least partially arranged within the winding interstitial space (12),
**characterized in that** the closed compartment (20) comprises an insulation fluid (16a) which is different from the insulation fluid (16b) comprised in the remaining insulating space (22) of the transformer (2), the insulation fluid (16a) comprised in the closed compartment (20) having a higher dielectric breakdown field strength than the insulation fluid (16b) comprised in the remaining insulating space (22).

2. Transformer (2) according to claim 1, **characterized in that** the insulation fluid (16a) in the closed compartment and the insulation fluid (16b) in the remaining insulating space (22) are in liquid phase, in gaseous phase, or form a two-phase system comprising a first part in liquid phase and a second part in gaseous phase.

3. Transformer (2) according to any one of the preceding claims, **characterized in that** the closed compartment (20) comprises an insulation gas (16a) which has a higher pressure than the insulation gas (16b) comprised in the remaining space (22) of the transformer (2).

4. Transformer (2) according to any of the preceding claims, **characterized in that** the insulation fluid , in particular the insulation fluid (16a) in the closed compartment (20), comprises a fluoroketone having from 4 to 12 carbon atoms.

5. Transformer (2) according to claim 4, **characterized in that** the fluoroketone has the general structure R1-CO-R2, wherein R1 and R2 are at least partially fluorinated chains, said chains being independently from each other linear or branched and having from 1 to 10 carbon atoms, and that the fluoroketone is a perfluorinated ketone or a hydrofluorinated ketone, in particular that the fluoroketone has 6 carbon atoms and preferably has the molecular formula C₆F₁₂O and most preferably is dodecafluoro-2-methylpentan-3-one.

6. Transformer (2) according to any one of the claims 1-3, **characterized in that** the insulation fluid (16; 16a, 16b) is selected from the group of SF₆, air at high pressure, midel, mineral oil, and silicone oil.

7. Transformer (2) according to any one of the preceding claims, **characterized in that** the windings (6, 8) are embedded in the material of the block (18).

8. Transformer (2) according to any one of the preceding claims, **characterized in that** the relative permittivity of the material of the block (18) is higher than the relative permittivity of the insulation fluid (16a) in the closed compartment (20), preferably by a factor of at least 1.5, and more preferably by a factor higher than 3.

9. Transformer (2) according to any one of the preceding claims, **characterized in that** the block (18) is made of an epoxy resin.

10. Transformer (2) according to any one of the preceding claims, **characterized in that** the closed compartment (20) is divided by at least one barrier (24) into at least two sub-compartments (26a, 26b).

11. Transformer (2) according to any one of the preceding claims, **characterized in that** it further comprises a pressure control system (32) designated to control the pressure in the closed compartment (20).

12. Transformer (2) according to any one of the preceding claims, **characterized in that** it further comprises a cooling system (28) for cooling the block (18), said cooling system (28) comprising means for circulating the insulation fluid (16a) in a closed circuit from the closed compartment (20) to an external space (30), where heat is released.

13. Transformer (2) according to claim 1, **characterized in that** the closed compartment (20) corresponds at least approximately to the winding space (12).

14. Transformer (2) according to any one of the preceding claims, **characterized in that** the electrical active part is enclosed by a tank and thus is arranged in a closed insulating space, or alternatively, the transformer (2) is devoid of a tank and the insulation fluid (16, 16b) in the remaining insulating space (22) outside the closed compartment (20) is air at atmospheric pressure.

15. Use of a transformer (2) according to any one of the preceding claims for operation at a voltage in the range from 10 kV to 300 kV, preferably from 20 kV to 150 kV, more preferably from 36 kV to 110 kV, and most preferably at about 72.5 kV.

## Patentansprüche

1. Transformator (2), der einen isolierenden Raum, der ein Isolationsfluid (16, 16b) umfasst, und einen elektrisch aktiven Teil enthält,
wobei der elektrisch aktive Teil in dem isolierenden Raum angeordnet ist und eine Primärwicklung (8), eine Sekundärwicklung (6) und einen magnetischen Kern (10) umfasst,
wobei die Wicklungen (6, 8) um den magnetischen Kern (10) herumgewickelt und voneinander radial durch einen Wicklungszwischenraum (12) getrennt sind,
wobei der Transformator (2) ferner einen Block (18) umfasst, der einen Raum (20) definiert, die von einem verbleibenden Isolationrsraum (22) geschlossen und abgetrennt wird, wobei der geschlossene Raum (20) mindestens teilweise innerhalb des Wicklungszwischenraums (12) angeordnet ist,
**dadurch gekennzeichnet, dass** der geschlossene Raum (20) ein Isolationsfluid (16a) umfasst, das sich von dem Isolationsfluid (16b), das in dem verbleibenden Isolationsraum (22) des Transformators (2) enthalten ist, unterscheidet, wobei das Isolationsfluid (16a), das in dem geschlossenen Raum (20) enthalten ist, eine höhere dielektrische Durchschlagsfeldstärke aufweist als das Isolationsfluid (16b), das in dem verbleibenden Isolationsraum (22) enthalten ist.

2. Transformator (2) nach Anspruch 1, **dadurch gekennzeichnet, dass** das Isolationsfluid (16a) in dem geschlossenen Raum (20) und das Isolationsfluid (16b) in dem verbleibenden Isolationsraum (22) in flüssiger Phase oder in gasförmiger Phase vorliegen oder ein Zweiphasensystem bilden, das einen ersten Teil in flüssiger Phase und einen zweiten Teil in gasförmiger Phase umfasst.

3. Transformator (2) nach einem der vorhergehenden Ansprüche, **dadurch gekennzeichnet, dass** der geschlossene Raum (20) ein Isolationsgas (16a) umfasst, das einen höheren Druck als das in dem verbleibenden Raum (22) des Transformators (2) enthaltene Isolationsgas (16b) aufweist.

4. Transformator (2) nach einem der vorhergehenden Ansprüche, **dadurch gekennzeichnet, dass** das Isolationsfluid, insbesondere das Isolationsfluid (16a) in dem geschlossenen Raum (20), ein Fluorketon mit 4 bis 12 Kohlenstoffatomen umfasst.

5. Transformator (2) nach Anspruch 4, **dadurch gekennzeichnet, dass** das Fluorketon die allgemeine Struktur R1-CO-R2 aufweist, wobei R1 und R2 mindestens teilweise fluorierte Ketten sind, wobei die Ketten unabhängig voneinander linear oder verzweigt sind und 1 bis 10 Kohlenstoffatome aufweisen, und dass das Fluorketon ein perfluoriertes Keton oder ein hydrofluoriertes Keton ist, insbesondere dass das Fluorketon 6 Kohlenstoffatome aufweist und vorzugsweise die Summenformel C₆F₁₂O aufweist und besonders bevorzugt Dodekafluor-2-methylpentan-3-on ist.

6. Transformator (2) nach einem der Ansprüche 1-3, **dadurch gekennzeichnet, dass** das Isolationsfluid (16; 16a, 16b) aus der Gruppe von SF₆, Luft mit hohem Druck, Midel, Mineralöl und Silikonöl ausgewählt ist.

7. Transformator (2) nach einem der vorhergehenden Ansprüche, **dadurch gekennzeichnet, dass** die Wicklungen (6, 8) in dem Material des Blocks (18) eingebettet sind.

8. Transformator (2) nach einem der vorhergehenden Ansprüche, **dadurch gekennzeichnet, dass** die relative Permittivität des Materials des Blocks (18) größer ist als die relative Permittivität des Isolationsfluids (16a) in dem geschlossenen Raum (20), bevorzugt um einen Faktor von mindestens 1,5 und besonders bevorzugt um einen Faktor größer als 3.

9. Transformator (2) nach einem der vorhergehenden Ansprüche, **dadurch gekennzeichnet, dass** der Block (18) aus einem Epoxidharz besteht.

10. Transformator (2) nach einem der vorhergehenden Ansprüche, **dadurch gekennzeichnet, dass** der geschlossene Raum (20) durch mindestens eine Barriere (24) in mindestens zwei Unterräume (26a, 26b) aufgeteilt wird.

11. Transformator (2) nach einem der vorhergehenden Ansprüche, **dadurch gekennzeichnet, dass** er ferner ein Drucksteuerungssystem (32) umfasst, das zum Steuern des Drucks in dem geschlossenen Raum (20) vorgesehen ist.

12. Transformator (2) nach einem der vorhergehenden Ansprüche, **dadurch gekennzeichnet, dass** er ferner ein Kühlsystem (28) zum Kühlen des Blocks (18) umfasst, wobei das Kühlsystem (28) Mittel zum Zirkulieren des Isolationsfluids (16a) in einem geschlossenen Kreislauf von dem geschlossenen Raum (20) zu einem externen Raum (30), wo die Wärme freigesetzt wird, umfasst.

13. Transformator (2) nach Anspruch 1, **dadurch gekennzeichnet, dass** der geschlossene Raum (20) zumindest annähernd dem Wicklungsraum (12) entspricht.

14. Transformator (2) nach einem der vorhergehenden Ansprüche, **dadurch gekennzeichnet, dass** der elektrisch aktive Teil von einem Tank umschlossen wird und somit in einem geschlossenen Isolationsraum angeordnet ist, oder dass der Transformator (2) alternativ keinen Tank aufweist und das Isolationsfluid (16, 16b) in dem verbleibenden Isolationsraum (22) außerhalb des geschlossenen Raums (20) aus Luft mit Atmosphärendruck besteht.

15. Verwenden eines Transformators (2) nach einem der vorhergehenden Ansprüche zum Betrieb bei einer Spannung im Bereich von 10 kV bis 300 kV, bevorzugt von 20 kV bis 150 kV, besonders bevorzugt von 36 kV bis 110 kV und ganz besonders bevorzugt bei etwa 72,5 kV.

## Revendications

1. Transformateur (2) contenant un espace d'isolation, qui comprend un fluide d'isolation (16, 16b), et une partie électrique active,
la partie électrique active étant disposée dans l'espace d'isolation et comprenant un enroulement primaire (8), un enroulement secondaire (6) et un noyau magnétique (10),
lesdits enroulements magnétiques (6, 8) étant enroulés autour du noyau magnétique (10) et étant espacés radialement l'un de l'autre par un espace interstitiel d'enroulement (12),
ce transformateur (2) comprenant en outre un bloc (18) définissant un compartiment (20) fermé et séparé d'un espace isolant restant (22), ledit compartiment fermé (20) étant disposé au moins partiellement à l'intérieur de l'espace interstitiel d'enroulement (12),
**caractérisé en ce que** le compartiment fermé (20) comprend un fluide d'isolation (16a) qui est différent du fluide d'isolation (16b) compris dans l'espace d'isolation restant (22) du transformateur (2), le fluide d'isolation (16a) compris dans le compartiment fermé (20) ayant une rigidité diélectrique plus élevée que le fluide d'isolation (16b) compris dans l'espace d'isolation restant (22).

2. Transformateur (2) selon la revendication 1, **caractérisé en ce que** le fluide d'isolation (16a) dans le compartiment fermé et le fluide d'isolation (16b) dans l'espace d'isolation restant (22) sont en phase liquide, en phase gazeuse, ou forment un système à deux phases comprenant une première partie en phase liquide et une deuxième partie en phase gazeuse.

3. Transformateur (2) selon l'une quelconque des revendications précédentes, **caractérisé en ce que** le compartiment fermé (20) comprend un gaz d'isolation (16a) qui a une pression plus élevée que le gaz d'isolation (16b) compris dans l'espace restant (22) du transformateur (2).

4. Transformateur (2) selon l'une quelconque des revendications précédentes, **caractérisé en ce que** le fluide d'isolation, en particulier le fluide d'isolation (16a) dans le compartiment fermé (20), comprend un fluorocétone ayant de 4 à 12 atomes de carbone.

5. Transformateur (2) selon la revendication 4, **caractérisé en ce que** le fluorocétone a la structure générale R1-CO-R2, dans laquelle R1 et R2 sont des chaînes au moins partiellement fluorées, lesdites chaînes étant, indépendamment l'une de l'autre, linéaires ou ramifiées et ayant 1 à 10 atomes de carbone, et **en ce que** le fluorocétone est un cétone perfluoré ou un cétone hydrofluoré, en particulier **en ce que** le fluorocétone a 6 atomes de carbone et a de préférence la formule moléculaire C₆F₁₂O et, ce qui le plus préférable, est du dodécafluoro-2-méthylpentan-3-one.

6. Transformateur (2) selon l'une quelconque des revendications précédentes 1 à 3, **caractérisé en ce que** le fluide d'isolation (16 ; 16a, 16b) est choisi parmi le groupe comprenant SF₆, de l'air sous haute pression, de l'huile Midel, de l'huile minérale et de l'huile de silicone.

7. Transformateur (2) selon l'une quelconque des revendications précédentes, **caractérisé en ce que** les enroulements (6, 8) sont encastrés dans le matériau du bloc (18).

8. Transformateur (2) selon l'une quelconque des revendications précédentes, **caractérisé en ce que** la permittivité relative du matériau du bloc (18) est plus élevée que la permittivité relative du fluide d'isolation (16a) dans le compartiment fermé (20), de préférence d'un facteur d'au moins 1,5, et, ce qui est plus préférable, d'un facteur supérieur à 3.

9. Transformateur (2) selon l'une quelconque des revendications précédentes, **caractérisé en ce que** le bloc (18) est fait de résine époxyde.

10. Transformateur (2) selon l'une quelconque des revendications précédentes, **caractérisé en ce que** le compartiment fermé (20) est divisé par au moins une barrière (24) en au moins deux compartiments secondaires (26a, 26b).

11. Transformateur (2) selon l'une quelconque des revendications précédentes, **caractérisé en ce qu'**il comprend un système de régulation de pression (32) désigné de façon à réguler la pression dans le compartiment fermé (20).

12. Transformateur (2) selon l'une quelconque des revendications précédentes, **caractérisé en ce qu'**il comprend en outre un système de refroidissement (28) pour refroidir le bloc (18), ledit système de refroidissement (28) comprenant un moyen pour faire circuler le fluide d'isolation (16a) dans un circuit fermé du compartiment fermé (20) à un espace externe (30), où la chaleur est dégagée.

13. Transformateur (2) selon la revendication 1, **caractérisé en ce que** le compartiment fermé (20) correspond au moins à peu près à l'espace d'enroulement (12).

14. Transformateur (2) selon l'une quelconque des revendications précédentes, **caractérisé en ce que** la partie électrique active est enfermée par une cuve et est ainsi disposée dans un espace d'isolation fermé, ou, en variante, le transformateur (2) est dépourvu de cuve et le fluide d'isolation (16, 16b) dans l'espace d'isolement restant (22) à l'extérieur du compartiment fermé (20) est de l'air à la pression atmosphérique.

15. Utilisation d'un transformateur (2) selon l'une quelconque des revendications précédentes destiné à fonctionner à une tension située dans la plage allant de 10 kV à 300 kV, de préférence de 20 kV à 150 kV, ce qui est plus préférable, de 36 kV à 110 k V, et ce qui est le plus préférable, à environ 72,5 kV.
